# EUROPEAN PATENT APPLICATION

(11) **EP 4 793 968 A1**
(43) Date of publication of application: **19.08.2026**
(21) Application number: 25165495.0
(22) Date of filing: 22.03.2025
(51) Int. Cl.: G16H 40/67, G16H 50/20, G16H 50/30

(54) **SELF-SERVICE TERMINAL FOR DIGITAL HEALTHCARE**

(30) Priority: 14.02.2025 BG 636725
(71) Applicant: EKSPERTITE Ltd, 5770 Lukovit (BG)
(72) Inventor: Lalev, Lyuben, Sofia (BG)
(74) Representative: Benatov, Samuil Gabriel

(57) **Abstract**

A self-service eHealth station according to the invention, includes a set of medical devices (1) for diagnosing and screening various medical conditions and diseases, a database memory (3) connected to a processing unit (5) that is connected to a user interface (7), and a logic unit (13). The station provides the capability for autonomous and highly efficient care, access to the patient's medical record and 10 real-time consultations with a health professional.

## Description

### Field of invention

The present invention relates to the field medicine, more particularly to a system for health care and remote health diagnostics.

### Prior art

Medical services are traditionally provided in doctors' offices or medical facilities. Usually people turn to their provider for medical care when they need treatment, and then a time and date are set for an examination. In many cases, however, these times are inconvenient for the patient. In addition, some patients may need more urgent care and cannot wait for the scheduled date.

In such situations, they often resort to emergency rooms or medical clinics, if available and easily accessible. In small towns and rural areas, medical facilities are often located at considerable distances, a visit inconvenient. Because of this, many people give up seeking medical care despite their need. Additionally, a visit to a health facility often requires high transport costs and significant transport time, as well as additional loss of valuable waiting time in front of the respective doctor's offices. An additional problem is related to global epidemics and increasing pressure on health systems, which often find themselves overwhelmed and underperforming in meeting large numbers of patients.

From the prior art are known medical procedure stations in which are installed a set of medical devices for diagnosis and screening of various medical conditions and diseases, a memory with a database connected to a processing unit that is connected to a user interface, and a power supply unit to provide power to the station. These stations do not provide sufficient autonomy for patient testing, and are not efficient enough with respect to the above problems.

In view of these challenges, there is a need for a new approach to medical service delivery that is more convenient, accessible, timely and cost-effective.

### Summary of the invention

The object of the present invention is to create an innovative system for medical services, diagnostics, health advice and wellness to individuals that is accessible from any, is for any age category, provides link to the patient's medical record, provide the opportunity for a wide range of medical examinations and tests, have increased efficiency regarding the examinations and tests performed, and provide convenient communication between patients and one or more medical providers.

This task is solved by creating the system according to the invention, being a self-service eHealth station comprising an enclosure in which are mounted a set of medical devices for testing, diagnosing and screening various medical conditions and diseases, a memory with a database connected to an information processing module with a data processor connected to a terminal for analyzing data for identification of the patient, and with the ability to receive query information from a user interface, and with the ability to receive and transmit data to and from database, and a power supply to provide electrical power to the station.

The set of medical devices includes at least two of stethoscope, otoscope, thermometer, spirometer, pulse oximeter, blood apparatus, scale, ECG, gynecological portable Doppler ultrasound, ophthamologic eye diagnostic system, thermal pad, magnifying glass, tongue depressor, forceps, blood glucose meter, bioresonance machine, bioimpedance machine, bone density densitometer, thermal camera, dermatoscope, body and/or muscle mass and/or fat index measuring apparatus, high resolution cameras, breath alcohol meter analyzer/tester, digital meter, tape measure, audiometer, fetal doppler, and the station also includes a monitor for data visualization.

The data processor is connected to a logic block comprising serially connected:
- patient identity verification module linked to the ability to receiving data from the patient identification terminal, which patient identity verification module is linked to the ability to enable or disable the activation of its subsequent modules based on an established patient identity,
- module for collecting patient vital signs data by medical devices in the station, from manually entered data via the user interface,
- a data preprocessing module configured with the ability to systematize the data collected by the data acquisition module and with the possibility of filling in missing values by statistical or other qualitative or quantitative methods,
- module for analysis and detection of patient health abnormalities and assessment, configured with the ability to analyze personalized information and general statistical information about conditions and diseases, to identify potential health problems by comparing a patient's current condition, as determined from data obtained from medical devices, with reference values and with the ability to classify the degree of health risk against the derived results,
- test diagnostic and module configured with the ability to analyze the systematized data from the data preprocessing module and from the module for the analysis and detection of abnormalities in a patient's health and risk assessment, and provide a preliminary diagnosis or a personalized recommendation based on the analyzed data to the user interface and to the database of the station.

The station may also include a wireless communication module that is coupled with the ability to exchange data with the station's database memory. The external central database may be, for example, an external central database of a patient's medical record at a healthcare institution, wherein the communication module is connected with the ability to exchange data with it. It is possible that the communication module is connected to a data exchange capability and to a system for real-time communication with a healthcare professional.

The data acquisition module can also be configured to receive data from the external central database via the wireless communication module link.

Preferably, the communication module is configured to wirelessly connect to an external central database containing general information about medical diseases and conditions, and the information collection module is configured to receive data from the external central database containing general information on medical diseases and conditions, and the patient health abnormality analysis and risk assessment module is configured with the ability to match general information on medical diseases and conditions with patient test data, and the diagnostic module is configured with the ability to issue a conclusion and/or recommendation based on the result of the comparison.

In a preferred embodiment of the station, the logic unit also includes a module for communicating with an external healthcare professional database associated with the ability to receive and send data to and from the data acquisition module.

In a preferred embodiment of the station, the logic block also includes a mental health assessment module configured to analyze currently sensed parameters from the voice and/or data input analyzers 22 installed in the station, to compare them to reference values, and to send a result of the analysis to the data preprocessing module.

Preferably, the station shall include a system to control infectious risks by at least one of a UV disinfection device, a hydrogen disinfection device or other fluid disinfection, and automatic ventilation.

Preferably, the station includes one or more apparatus storage chambers, tests or other material with limited access via a locking means, configured to be unlocked manually or electronically by remote means via a transceiver module connected to the locking means.

Preferably, the user interface is connected to a payment module with data exchange capability.

In the preferred version of the station implementation, the diagnostics module in the logic block is connected to a health prediction module configured with the ability to predict future health risks using machine learning algorithms based on data from the diagnostics module. Preferably, the station is mobile.

Preferably, the station includes a patient notification system for accessibility of the station, for a control review or a finished prescription, comprising a notification module coupled to the processor and the station database, which notification module is configured to send data to an external device wirelessly, or to an indicator device of the station.

Preferably, the notification module in the station is configured to send and receiving information to and from an external mobile device.

Advantages of the station are:
Providing at accurate diagnoses, personalized plans about treatment and advanced monitoring; Carrying out an independent health check by telemetry;
Avoiding restrictions on patient or doctor mobility and waiting times for medical tests as well as examinations and consultations.

Provides a positive effect on the productivity of a company where an employee works, while saving time and providing objective outcomes of employees wishing to go on "unauthorized sick leave" or "faking" illness. As well as prevention for employees who are subject to mandatory daily health assessment before admission to the, e.g. crane operators, excavators, drivers, operators, NPP employees, etc. The assessment is objective, impartial, documented, archived on file (by date, time, video) and can be signalled to a doctor for urgent care, which is another unique advantage;
Improving the autonomy of older people;
Support the work of national health institutions to collect data to prevent further complications in time;
Optimizing the allocation of health resources and reducing the workload of hospital facilities;
Facilitating access to health services through modern technology;
Useful in epidemic crises as it minimises the need for patients to visit where the risk of infection is high.

It is an effective tool for early diagnosis, testing and monitoring of diseases on a large scale;
Patients can connect in real-time with health professionals (medical or wellness specialists) via high-quality video and audio connection;
Patients can receive personalized healthcare services with minimal time to wait;
Improving the quality of life of citizens through easy access to health services;
Ensure flexibility and adaptability of the health system in emergency and unforeseen circumstances;
Creating a sustainable platform for the future development of eHealth;
Ability to work both autonomously and with the involvement of an assistant who be a medical or non-medical person;
Protecting patient privacy due to limited access to information through a card verification system, ID, SSN or facial recognition, or other means/method of identifying an authorized person or the patient;
Providing an intuitive interface that is easy to use for all ages and persons with any technical knowledge;
Delivering customized health reports generated using artificial intelligence logic block.

### Brief description of the figures

Later in the description, the eHealth self-service station is explained by way of a preferred, given as a non-limiting example within the scope of the invention, with reference to the accompanying figure, wherein:

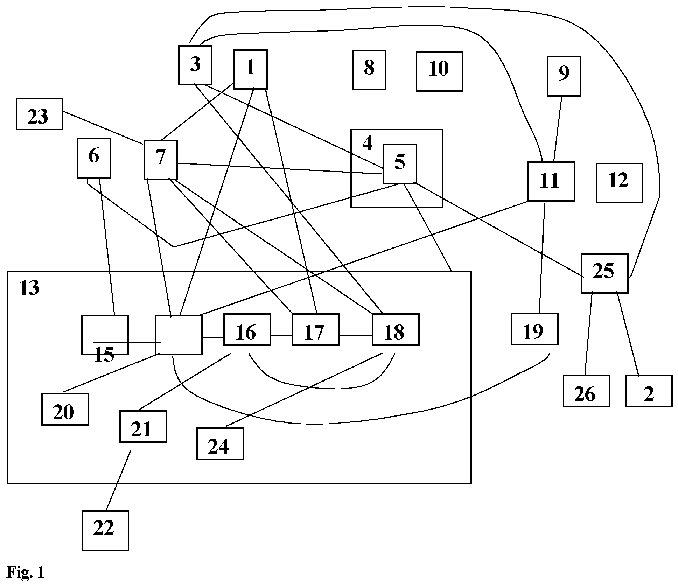

FIG. 1 is a block diagram of a preferred embodiment of a self-service eHealth station according to the invention.

### Exemplary embodiments of the invention

The eHealth Self-Service Station, is a high-tech, autonomous healthcare service delivery solution combining eHealth consultation, diagnosis and real-time monitoring.

The eHealth Self-Service Station operates on the principle of eHealth, which uses modern information and communication technologies to deliver health services and medical care remotely without the need to be physically present in a health facility, thereby facilitating access to health services. Thus, remote healthcare consultations with a health specialist (medical or wellness specialist), provide the opportunity for monitoring and diagnosis of health status through the use of medical devices 1 for monitoring physiological indicators, and provide the opportunity to monitor the condition of patients, as well as to issue recommendations for improving the health status and treatment of various ailments and diseases. All test and monitoring results as well as recommendations can be issued in two ways: by direct contact with a health professional or by analysis from logic block 13 and rapid delivery of the result.

As shown in Figure 1, the station is a self-contained structure comprising a housing in which are mounted a set of medical devices 1 for testing, diagnosing and screening various medical conditions and diseases, a memory 3 connected to an information module 4 with a data processing unit 5 that analyzes data from a patient identification terminal 6, receives requests for information from user interface 7, and receives and transmits data to and from database 3. The station also includes a power supply unit 8 for providing electrical power to the station, a communication system 9 for wireless audio or audio and video communication with a health care professional, a monitor 10 for displaying data, a communication module 11 for wireless connection to an external central database 12 with a medical record of a patient in a health care institution, and a logic block 13 connected to the data processing unit 5. The communication unit 11 for wireless connection to an external central 12 with a medical record of a patient in a health care institution is connected to the station's database memory 3 and to the real-time communication system 9 with a health care professional, and exchanges data therewith.

The information module 4 with the station processor 5 has the following functions:
- receives requests for information from the user interface 7,
- queries and accepts data to/from the station's local database 3,
- sends visualization data to the monitor 10,
- communicates with an external central database 12 via the logic block 13, by querying and accepting data from this external central database 12. In various embodiments of the invention, the logic block 13 may only access information from the external central database 12, or alternatively and send information to it.

The equipment in the medical station is used to store one or more medical devices 1, and may include, some or all of the following devices 1: a stethoscope, a thermometer, a spirometer, a pulse oximeter, a blood pressure apparatus, a scale, an ECG, an ophthalmic eye diagnostic system, a heat pad, a magnifying glass, a tongue depressor, a forceps, a blood glucose meter, a bioresonance apparatus, a bioimpedance apparatus, a measuring tape, a bone densitometer, Gynaecological portable Doppler ultrasound, thermal imaging camera, dermatoscope, body and/or muscle mass index and/or fat measurement device, high resolution cameras, breath alcohol analyser/tester, digital meter, audiometer, otoscope, fetal Doppler, monitor. It is also possible to use additional apparatus 1. Each of the apparatuses 1 may be configured to send measured result data via the central station system to a remote medical person, for example, via a connection to the central station system via a wired path or a wireless path, for example Bluetooth, Wi-fi, VPN control.

Health parameters that the health station can measure are for example:
1. A general health examination, which may include some, all or several health parameters of individuals:
   - Oxygen saturation
   - Pulse
   - Body temperature
   - Height
   - Body weight
   - Body mass index (BMI)
   - BMR (fat mass index)
   - Total body water content
   - Skeletal mass / Bone density
   - Muscle mass
   - Bone mass
   - Protein levels
   - Metabolic age
   - Fat free weight (FFM)
   - Total body fat percentage
   - Energy consumption at rest
   - Basic metabolism
   - Subcutaneous fat
   - Visceral fat
   - Physique type
   - Physical rating
2. Diagnosis of the respiratory system
   - Spirometry
   - Oximetry
   - Peak expiratory flow (PEF) measurement
   - function: FVC (Forced Vital Capacity)
   - Expiratory Air Volume: FEV (Forced Expiratory Volume)
   - FEV1/FVC ratio
3. Measurement of hemoglobin
4. Measure blood glucose, glucose, HBA1C levels
5. Hearing screening and diagnosis
   - Audiometry
   - Otoscopy
6. Cardiovascular system
   - ECG
   - Blood pressure measurement
      - Systolic pressure
   - Diastolic pressure
      - Mean arterial pressure
   - Pulse rate
   - Measurement and analysis of total cholesterol (TC), triglyceride ratios (TG), high density lipoprotein (HDL), low density
      lipoprotein (LDL) and TC/HDL ratio.
   - Blood pressure measurement
   - Blood pressure index
   - Arterial elasticity
   - Arterial stiffness
   - Heart rate
   - Maximum heart rate
   - Heart rate variability
7. Rapid tests - express diagnostic tests for rapid detection of various health conditions
8. Pregnancy monitoring - fetal health monitoring by fetal Doppler and cardiotocograph (CTG).
9. Mental health - a comprehensive assessment of psychoemotional status, including stress levels, burnout syndrome, depressive states and other mental factors affecting working capacity and general well-being.
10. Stethoscopy
   - Auscultation of the lungs and chest
   - Cardiovascular system research
11. Eye test
   - Diagnosis of myopia
   - Diagnosis of farsightedness
   - Color blindness test
   - Assessment of astigmatism
12. Dermatological assessment - review of skin status and skin
13. Breath Alcohol Analyzer/Tester - measure blood alcohol concentration
14. Other indicators
15. The station is equipped with telecommunication technologies, in particular the communication system 9 for wireless audio or audio and video connection, which allows video consultations with health professionals and real-time data transmission to health centers and medical analysis platforms.

This communication system 9 connects the patient and the healthcare, and in necessity sends alarms or reports to health care professionals about the patient's condition, presenting medical information in a language that the patient can understand via the user interface 7 and data display monitor 10. The communication system may include a camera, a monitor, a microphone, a speaker, and a . All transmitted information is encrypted and
protected against unauthorised access. Automated checks are performed for compliance with GDPR (General Data Protection Regulation), ISO 27001 (Information Security Management Systems), ePrivacy Directive (Cookie Law) and HIPAA (Health Insurance Portability and Accountability Act in the United States), or other regulations.

Logic block 13 includes the following modules connected in series:
- A patient identity verification module 14 that receives information from the patient identification terminal 6, for example by reading a patient ID or inputting personal data from the patient, and based on the result allows activation or disallows activation of the subsequent process in logic block 13;
- A data acquisition module 15 that collects and validates inputs from medical sensors and devices 1 at the station, from patient input via the station's user interface 7, from a history of health status obtained through integration with electronic health records. The algorithm filters and validates the data, removing erroneous or anomalous values;
- Data Preprocessing Module 16, which prepares raw data for analysis by bringing various measurements into a compatible format, fills in missing values by statistical or other qualitative or quantitative methods, e.g. interpolation, and labels the data (e.g. classifying risk indicators);
- Patient health abnormality analysis and detection and risk assessment module 17 that identifies potential health problems in a patient's current state using an algorithm with reference values for deviations from the norm of given vital signs, for example, using K-means clustering to group data or Decision Trees to analyze risk values, or using recurrent neural networks (RNNs) to detect anomalies in a time series. The module assesses risk through a system scoring system that classifies risk (low, medium, high) according to the abnormalities detected, and a notification is sent to the health professional when a risk is detected;
- An 18 test diagnosis and conclusion module that generates a preliminary diagnosis or recommendation using a trained model on medical data. The model analyses combined indicators to suggest possible diagnoses. If images are available (e.g., ECG), a computer vision algorithm (CNN) analyzes the images. Finally, a personalized conclusion is issued based on the patient's history and the algorithm offers personalized recommendations. Recommendations are compiled according to the individual patient characteristics, offering personalised advice on diet, physical activity or lifestyle, with the algorithm updating recommendations as the patient's health status changes.
- Mental Health Assessment Module 21, which analyses mental condition through behavioural data and questionnaires. Textual analysis is used through an NLP algorithm that processes the patient's textual responses to detect signs of distress or depression, and voice analysis that uses an audio analysis algorithm to identify emotional changes.
   Module 21 classifies the risk of mental disorder and suggests next steps;
- A health condition prediction module 24 that predicts future health risks through machine learning algorithms, such as logistic regression or another algorithm predicting the likelihood of development of chronic diseases, or, for example, through LSTM models that analyze time series of indicators to predict conditions such as diabetes or hypertension;
   It is possible for a patient to have tests performed in an external laboratory, and then enter the results of these tests into the data collection module 15 via station's user interface 7, or alternatively, the results could be retrieved from an external database containing the patient's medical record, 17 then these results could be analyzed in the patient health abnormality analysis and detection and risk assessment 17 module, and a preliminary diagnosis or recommendation could be generated in the diagnostic testing and issuing a conclusion 18 as well as to predict future health risks through machine learning algorithms in the health condition 24 prediction module.

Logic Block 13 can provide accurate diagnoses, personalized treatment plans, and advanced monitoring. Specifically, the logic block 13 has the following features:
- through the logic block 13 algorithms, data is analyzed, anomalies are identified and alerts are sent to health professionals. This enables early diagnosis and intervention, preventing complications;
- Processes the patient's medical history and current data to create individualized plans to improve health status, rehabilitation, or treatment;
- Offers automated lifestyle, dietary and medication recommendations based on a patient's unique characteristics;
- Analyzes medical images, lab results and symptoms entered by patients;
- speeds up the diagnostic process and improves accuracy, reducing the risk of errors;
   - monitors mental health through questionnaires, voice analysis and behavioural data;
- detects early signs of depression, anxiety or other mental health conditions, offering guidance on consulting a specialist;
- processes the data collected from patients in real time and sends it to the medical team through secure channels;
   - enables constant monitoring of patients' health status, even when they are outside medical facilities.

Each station can access all health data of the respective patient, thus providing full access to his medical record, after the respective authentication of the said patient through a verification device, connected to the patient identity verification module 14, which may, for example, by ID, fingerprint, password or otherwise. The verification device may also be connected to a biometric capture device with data exchange capability.

All analyses performed at the station can be sent via communication module 11 over a wireless connection to an external central database 12 with a patient medical record at a health care institution containing a centralized health or medical record that can be accessed by national, supranational, or other private, public, and governmental institutions, as well as statistical institutions and organizations. This avoids abuse of sick notes and the's medical record is completed. This linkage also enables online prescriptions and referrals to be made available to the relevant centralised systems through which prescriptions are filled and the patient's health record kept up to date. This in turn facilitates the production of correct statistics, for example in order to derive demographic data, as well as monitoring trends in morbidity and the ability to anticipate possible risks of future epidemics, and to establish timely preventive solutions in accordance with established national and regional health policies.

It is possible that the logic block 13 also communicates with another external central database 19, which includes information about medical diseases and conditions, the logic block 13 analyzes the patient's examination results and retrieves similar results from this external central database 19, and thereby analyzes and provides any recommendations or conclusions about the's condition.

This connection is made through the following modules:
- The communication module 11, which provides a wireless connection to an external central database 19,
- the information collection module, which receives data from the external central database 19 containing general information on medical
   diseases and conditions,
- the Patient Health Abnormality Analysis and Detection and Risk Assessment Module 17, which correlates general information about medical diseases and conditions with patient test data,
- the diagnostic module 18, which issues a conclusion and/or recommendation based on the result of the comparison.

By means of a communication module with an external database of the health professional 20, the logic block 13 provides a connection between the station's database 3 and the database of the respective health professional, thereby ensuring that the patient's data and test results at the station are linked to the record that
is taken to the relevant health professional who treats him.

It is possible to generate information about side effects of a medication, a description of a procedure, test or drug, health professional or treatment reviews, information pertaining to patient compliance with treatment, maintenance recommendations, generic alternatives to expensive medications, reminders to take medications, schedule a health professional, discount medication offers from pharmacies, and other information.

The information module 4 may additionally be connected to an external device by wireless electronic means, for example to a mobile device, on which messages may be displayed and through which requests for information may be made by a user (patient or health professional).

All data can also be stored on cloud space by connecting to the communication module 11. The database 3 of one station is connected via its processor 5 to the processors and databases 3 of all other similar stations located at different geographical locations. The connectivity is achieved by means known in the prior, wirelessly or wired.

The station may also include a translation module connected to the user interface 7 and the information module 4, through which each written message and each information to be translated into another language. It is also possible to connect the translation module the communication system 9 so that it can perform real-time speech translation in consultation with a health professional.

The health station can be used by the patient to enter or transmit basic information such as: patient name, address, contact information with the patient (e.g., home and work address, phone number, email address, pager number, work number, etc.), 's age, 's gender, patient's height, patient's weight, patient's medical history, current medications patient is using, reasons for patient's visit, 's current symptoms, 's insurance information, patient's intent to pay, patient's current physician, guardian or parent information, next of kin information, preferred medical provider for the visit, allergy information, previous visit information, medical record and/or pharmacy information, other health or medical needs related information.

In certain cases, the self-service station includes one or more storage chambers, for example of equipment, which may, for example, be located on or near the interior walls of the health station. These chambers may also be located in other areas of the station.

Storage chambers can also be used to store other materials such as bandages, gauze, cotton balls, disposable glucometer, disinfectant wipes and other medical supplies. The door of these chambers usually restricts access. It can be opened or closed manually by the assisting staff or/and remotely by the assisting staff or health professional by sending a command electronically from an electronic device (external mobile device, computer external to the station, user interface 7) via the processor 5 to a transceiver module connected to the locking means of the respective storage chamber.

The station is designed with a modern and futuristic design, providing an intuitive and accessible experience for users (patients). The self-service station for eHealth can vary in size, configuration, tilt and design, and these features do not limit its functionality. The station provides a private or semi-private environment for communication. This is provided by the design options of its housing, which may be fully open to the exterior, or be a special enclosure of panels framing walls, and optionally a door and roof, of the station that allows the patient to enter, sit, and interact with the medical provider discreetly. The enclosure may be composed of one or multiple walls, providing the necessary protection and privacy to users. These walls may be fully or partially enclosed, to create a comfortable environment for interaction. The enclosure can be designed in different shapes, sizes and configurations depending on the needs and space. Different variations are possible, and there may be medical or non-medical personnel inside or in the area of the enclosure, depending on the need as well as the situation for which the invention is used. It is possible that the housing to be equipped with a ramp for the disabled, for access by persons in a difficult position. This approach provides a new level of accessibility and comfort for patients, making it easier to connect with healthcare professionals regardless of their location. The ergonomic design includes a comfortable seat or chair, and in some cases a bed, for example when there is an attendant or medical person present, and easy-to-use touch screens.

The station walls may be soundproofed to ensure privacy during medical consultations. The station also houses lighting, electrical outlets that can also have USB inputs and outputs. The user interface 7 of the station includes one or more patient data entry terminals, which may be located outside the station or inside the station, or may be embedded within the station housing.

Personal data or data indicating the results of medical tests may be entered by a user through the data entry terminals, such as for example, a rapid flu test, a pregnancy test, or another type of test. Said medical (Rapid) tests are stored in dedicated locations inside the station, for example in storage chambers or to be stored in an additional vending module located outside the medical station, integrated in housing, connected to a data exchange capable payment module 23.

The payment module 23 is connected to the user interface 7, and provides options for different types of payments.

Data entry terminals may include touchless buttons to provide additional hygiene and security.

Data entry terminals may include any or all of the following devices:
- Monitor 10 to display information related to identification and/or data entry;
- Camera and/or video camera to collect identification information and/or entry;
- Touch keyboard, button keyboard, or keyboard board for the same;
- Touch screen for identification and/or entry;
- Microphone and recognition software;
- scanner;
   - Retinal scanner;
- Face and/or body scanners for the same purposes.

Other devices may be included in the station for displaying or collecting information related to identification and/or data entry.

It is also possible that the health station has a patient notification system, that the station is available (available for access), unavailable (occupied by another patient), or will soon be available. The notification may be sent by a notification module 25 coupled to the patient verification module 14 and to the diagnostic test and conclusion module 18, which notification module 25, when establishing verification, reports that the station is occupied, and upon completion of the diagnostic, determines that the station is vacant. Alternatively, the notification module 25 may be coupled to a motion sensor in the station, or at the entrance to the station, to detect presence in the station. The notification module 25 sends data (station occupancy or vacancy signal) to an external mobile device 26, or to an indicator device 2 of the station. The indicator device 2 may be light and/or sound indicators as well as digital displays that provide information as to whether the station is in use or available. Wireless notification to an external mobile device 26, e.g., a user's mobile phone, may be via email, text, phone call, SMS, email, or by other electronic means via the internet, such as a mobile app to a patient's mobile device.

The notification system can also be used to inform the patient when and/or where other stations are available. This service, when, can be used to inform the patient that another nearby station has a shorter waiting period or is currently available, thus providing the patient with the option of traveling to another available station rather than waiting for the current medical station to become available. The notification system can also be used to inform the patient when his prescription is ready for receipt, and/or to transmit prescription information to the patient. In, it can also be used to inform the patient when a follow-up visit is upcoming or scheduled.

The eHealth self-service station may include a remote account management module coupled to the station's processor 5 and to the logic block 13, through which a module can be used to customize the language of operation at the station, to schedule appointments for visits to the station, and to consult with specific health professionals or request specific medical tests to be delivered to the station prior to the patient's visit. This module can be accessed by both the patient and the assisting staff or health professional.

The eHealth self-service station may be designed to provide information to the patient before and/or during the input/transmission of information from the patient to the medical station. The station may include audio and/or visual instructions and/or displays that are part of information modules connected to the processor 5 and logic block 13 of the station, and generating messages used to provide information about the health station, how to use the health station, how to properly enter/submit information to the health station, instructions and/or interactions with the patient during the entry/transmission of information from the patient to the software at the eHealth station, patient wait times for use of the health station, a waiting list of patients for use of the health station, available medical providers, types of medical problems that can be resolved through use of the health station, insurance providers that can be used to partially or fully pay for a health visit, payment options for use of the health station, information on when the health station will be available in the future, information on whether the health station is in use or available.

The station may also include a medical result reporting module, which is connected to the data acquisition module 15, and to an analyzing device with a scanner for reporting a test result, in which case the patient health and risk assessment anomaly analysis and detection module 17 analyzes the reported result, and the diagnosis module 18 issues a conclusion about the test result.

The eHealth station may include a cleaning designed to clean the interior of the invention and/or remove/neutralize some or all of the microbes and/or other microorganisms in the medical station. Other or additional cleaning systems may also be used.

For example, the station may have a built-in infectious risk control system, including UV disinfection, hydrogen disinfection, or other fluid and automatic ventilation devices that provide a safe environment for each user. The devices of the infection control system may be made of high-strength plastics and/or other sufficiently rigid and strong materials, such as metal, and be constructed in such a that they cannot be manipulated by patients or others who are closer to them.

The station may also include flavoring apparatus, hazardous waste storage containers, and emergency kits including diagnostic apparatus, which may be connected via logic block 13 and communication module 11 by wireless path to the station's communication 9 system and database 3 or cloud space so as to record results of research performed. The emergency kit may also include medical diagnostic tests and a means for entering patient condition data and test results associated with the station or cloud space database 3.

The station may be made of one or more materials that are resistant to the spread of bacteria, viruses and/or other microorganisms.

The station can also be built on a modular type to allow it to be partially or fully disassembled into modules, or partially or fully folded. The modular construction of a health station may allow it to be deployed in different configurations, making it suitable for use in different types of spaces. In addition, the modular construction can be designed to facilitate the relocation of the medical station into an existing facility and allow it to be installed without having to modify the facility's entrances.

The station can be made of various materials, such as plastic, foam, metal, wood, composite materials and other materials. More specific examples of performance materials are, for example: thermopane panels, acoustic panels, thermo-foam, plexiglass, aluminum composite panels, etalbont, or other materials.

The invention contemplates the integration of diagnostic procedures, via instrumentation, medical tests, or the delivery and recovery of non-invasive test kits in a non-assisted medical station. It is intended for use in locations where medical services are not traditionally provided, including remote areas with limited access to medical, or in public spaces such as airports, shopping malls, universities, government institutions and corporate offices, as well as educational institutions, industrial zones and even in areas affected by natural disasters or epidemics, providing quick and convenient access to real-time medical care. Its application is particularly useful in areas with limited access to healthcare services or where necessary from mass health screenings and prevention campaigns.

The self-service station can be mobile, relocatable or fixed location.

### Exemplary operation of the station

The method of operation of the station described herein is provided as not limiting the scope of protection of the invention, and it is possible add additional stages, or eliminate some of the described stages, in operating the station subject to the invention.

First, patient registration is performed at the self-service station for eHealth via the user interface 7 or remotely from an external mobile device via the station's notification 25 module through several steps adapted to the patient's needs and the healthcare facility's requirements. The registration system provides options that are optional and not mandatory, such as entering allergy information, entering information on medical conditions, entering information on medications taken, entering medical insurance information, entering payment method information.

Once registration is complete, the system can provide a list of available time intervals for visiting the medical station. These intervals can be fixed over a period (e.g. every 20 or 30 minutes). The booked appointment time is then confirmed and sent to the patient.

To use the station, the patient enters the station equipped with measuring devices, verifies their identity, and follows various on-screen instructions.

The functionalities allow, for example, measuring blood pressure (blood pressure machine) and blood oxygen saturation (pulse oximeter), checking weight, height and temperature, testing visual acuity and hearing, conducting an electrocardiogram, and finally video-linking with a doctor. It takes only a few to collect and analyze the data, and the results are immediately sent to the medical record in the appropriate central database 12 of a health care institution, as well as to the station's database 3 and to the physician associated with the patient. The health professional may also request additional tests if necessary. Finally, the patient receives a result and an appointment for treatment, which is also entered in his medical record. The medical assistant or station attendant (assistant) can provide assistance with payment or another step in the process. The station may include a button to request assistance if the patient encounters difficulty.

During the patient's self-autonomous examination, the chamber door can be opened and/or unlocked by the assisting staff. After the patient leaves the medical station, if there is a medical/non-medical assistant staff may be able to come in after the 's examination is complete and clean the equipment that was used and/or discard and replace items that were used by the patient. Staff can then replenish, replace or rearrange medical equipment, in cabinets and close doors before the next patient enters if necessary.

The reference numbers of the technical features are included in the claims for the sole purpose of increasing the intelligibility of the claims and, therefore, these reference numbers do not have any limiting effect on the interpretation of the elements designated by these reference numbers.

## Claims

1. A self-service eHealth station comprising an enclosure in which are installed a set of medical devices (1) for testing, diagnosis and screening of various medical conditions and diseases, a memory with a database (3) connected to an information module (4) with a data processing unit (5) connected with capability of data analysis of a patient identification terminal (6), and with the capability of receiving query information from a user interface (7), and with the capability of receiving and transmitting data from and to the database (3), a power unit (8) for providing electrical power to the station, wherein the set of medical devices (1) includes at least two of a stethoscope, an otoscope, a thermometer, a dermatoscope, a spirometer, a pulse oximeter, a blood pressure apparatus, a weight scale, an ECG, a gynecological portable Doppler ultrasound, an ophthamological diagnostic system, a heat pad, a magnifying glass, a tongue depressor, a forceps, a blood glucometer, bioresonance device, bioimpedance device, bone density densitometer, thermocamera, body and/or muscle mass index and/or fat measurement device, high resolution cameras, respiratory alcohol analyzer/tester, digital meter, tape measure, audiometer, fetal doppler, and the station also includes a monitor (10) for visualizing data **characterizing in that** the data processing unit (5) is connected to a logic block (13) comprising serially connected:
- a patient identity verification module (14) connected with a capability of receiving data from the patient identification terminal (6), which patient identity verification module (14) is connected with a capability of activating or disabling the activation of its subsequent modules based on an established patient identity,
- a data collection module (15) from patient vital signs measurements via the medical devices (1) in the station, and from manually entered data via the user interface (7).
- a data preprocessing module (16) configured with the ability to systematize the collected data by the data collection module (15) and with the ability to fill in missing values using statistical or other qualitative or quantitative methods,
- module for the analysis and detection of abnormalities in a patient's state of health and risk assessment (17), configured with the ability to analyze personalized information and general statistical information on conditions and diseases, to identify potential health problems by comparing a patient's current status, established based on the data obtained from the medical devices (1), which are entered in the user interface (7), with reference values, and with the ability to classify the degree of health risk against the derived results,
- a module for diagnostic tests and issuing a conclusion (18) configured with the ability to analyse systematised data from data preprocessing module (16) and from the patient health abnormality analysis and detection and risk assessment module (17), and providing a preliminary diagnosis or personalized recommendation based on the analyzed data to the user interface (7) and to the database (3) of the station.

2. A self-service eHealth station according to any one of the preceding claims, **characterized in that** the station further comprises a communication module (11) for wirelessly connecting to an external central database, which is coupled to a data exchange capability with a database memory (3) of the station.

3. A self-service eHealth station according to claim 2, **characterized in that** it further comprises a communication system (9) for wireless audio or real-time audio and video communication with a healthcare professional, wherein the communication module (11) for wireless communication is coupled with the capability of data with the communication system (9) with a healthcare professional in real-time.

4. A self-service eHealth station according to any one of claims 2 and 3, **characterized in that** the communication module (11) is configured for wireless communication capable of exchanging data to an external central database (12) with a medical record of a patient at a healthcare institution, wherein the data acquisition module (15) is configured with the capability of receiving a data from the external central database (12) via the communication module (11) for wireless connection.

5. Self-service eHealth station according to any one of claims 2 to 4, **characterized in that** the communication module (11) is configured for wireless communication with another external central database (19), containing general information on medical diseases and conditions, and the data acquisition module (15) is configured to receive data from the external central database (19) containing general information on medical diseases and conditions, and the health anomaly analysis and detection module patient condition and risk assessment (17) is configured with the ability to match general information about medical diseases and conditions with patient test data, and the diagnosis module (18) is configured with the ability to issue a conclusion and/or recommendation based on the result of the matching.

6. A self-service eHealth station according to each of the preceding claims, **characterized in that** the logic block (13) further comprises a module for communicating with an external database of a healthcare professional (20) associated with the ability to receive and send data to and from the data acquisition module (15).

7. A self-service eHealth station according to each of the preceding claims, **characterized in that** the logic block (13) further comprises a mental health assessment module (21) configured to analyze currently sensed parameters from analyzing devices (22) for voice and/or input data installed in the station, to compare them with reference values, and to send a result of the analysis to the data preprocessing module (16).

8. A self-service eHealth station according to any one of the preceding claims, **characterized in** it comprises a system for infectious risks by at least one of a UV disinfection device, a hydrogen disinfection device or disinfection by another fluid, and automatic ventilation.

9. Self-service eHealth station according to any one of the preceding claims, **characterized in that** it comprises one or more chambers for storing apparatus, tests or other material with restricted access via a locking means configured to be unlocked manually or electronically remotely via a transceiver module connected to the locking means
means.

10. Self-service eHealth station according to any one of the preceding claims, **characterized in that** the user interface (7) is connected to a payment module (23) with data exchange capability.

11. A self-service eHealth station according to each of the preceding claims, **characterized in that** a health condition prediction module 24) configured to predict future health risks via machine learning algorithms based on data from the diagnosis module (18) is connected to the diagnosis module (18) in the logic block (13).

12. A self-service eHealth station according to any one of the preceding claims, **characterized in that** it is mobile.

13. A self-service eHealth station according to any one of the preceding claims, **characterized in that** it comprises a system for notifying the patient of the availability of the station, of a check-up or of a ready prescription, comprising a notification module (25) coupled to the processor (5) and database (3) of the station, said notification module (25) configured to send data to an external mobile device (26) , and/or to an indicator device (2) of the station.
